# EUROPEAN PATENT APPLICATION

(11) **EP 0 974 640 A1**
(43) Date of publication of application: **26.01.2000**
(21) Application number: 99304830.5
(22) Date of filing: 21.06.1999
(51) Int. Cl.: C11D 3/382, C11D 1/12, A61K 7/02

(54) **High lathering grain based cleansing bars**

(30) Priority: 22.06.1998 US 102439
(71) Applicant: JOHNSON & JOHNSON CONSUMER COMPANIES, INC., Skillman, NJ 08558 (US)
(72) Inventor: Leifheit, David H., Racine, Wisconsin 53406 (US); Rees, Wayne M., Racine, Wisconsin 53406 (US); Buri, David M., Union Grove, Wisconsin 53182 (US)
(74) Representative: Mercer, Christopher Paul

(57) **Abstract**

The disclosure provides processed grain based cleansing bars. Alkyl ether carboxylates are added to the bar to improve lathering. The bar is buffered so as to have a skin pH and includes synthetic surfactants such as isethionates.

## Description

### Background Of The Invention

The present invention relates to processed grain based cleansing bars having improved lathering characteristics. More particularly it relates to formulations of such bars which provide desirable cleansing characteristics at skin pH, without the need for high soap content.

"Soaps" are salts of fatty acids with various bases. Examples are the salts of fatty acids with ammonia, low molecular weight amines (especially alkanolamines) and alkali metals (especially sodium and potassium). Other fatty acid salts result from reaction with metallic cations (e.g. zinc and aluminum, alkaline earths such as calcium and magnesium), and long chain fatty amines. For hundreds of years soaps have been formed and used in the form of bars.

While soap bars are very effective at cleaning, they tend to dry skin by stripping away natural oils. Sometimes they also otherwise adversely affect skin because their pH is more basic than the skin. Soap bars can also leave undesirable "soap scum" in sinks. There have therefore been efforts to develop cleansing bars that do not incorporaLe soaps apart from the level of soap present in other desired ingredients (e.g. their storage medium).

Particularly desirable cleansing bars are processed grain based bars such as those described in U.S. patent 5,691,287. The disclosure of this patent is incorporated by reference as if fully set forth herein. While grain based cleansing bars have a number of advantages over traditional soap bars, consumers familiar with such bars have shown concern about the relatively low level of lathering of such bars. A foam enhancer, Lauramide DEA, was incorporated into these bars. However, Lauramide DEA did not adequately address this consumer concern.

Complicating matters is the fact that certain other lathering enhancers require high pH to work (and it is desirable to formulate grain based cleansing bars so that they have a skin pH). Other lathering enhancers are incompatible with certain grains. Still others interfere with desirable additives, or lead to cracking or the bars becoming overly soggy.

Thus, a need exists for a grain based cleansing bar having improved lathering characteristics at skin pH.

### Brief Summary Of The Invention

In one aspect the invention provides such a cleansing bar. It has at least 30% by weight of processed grain, at least 10% (preferably 20-40%) by weight of a surfactant which is not a soap, and at least .5% by weight of a lathering enhancer of the formula:

R-O-(CH₂CH₂O)ₙ-CH₂-COOH,

or a salt thereof, where R is an alkyl or alkylaryl moiety having between 6 and 20 (preferably 8-14) carbons and n is 1-20.

We define processed grain throughout this application to include, by way of example and not limitation, powdered grain, defatted grain, grain starch, oil-extracted grain, bleached grain, soluble grain fiber, grain protein, grain hulls, grain kernels, and grain bran. Any suitable grain may be employed, including, for example, oat, wheat, rice, barley, and corn. Preferred examples include oatmeal, oat flour, corn flour, oat starch, corn starch, and defatted microporous oat fraction.

We prefer to include colloidal oatmeal in our cleansing bar. It acts as a skin protectant to provide a barrier which helps prevent the skin from losing moisture. It also serves as a binder to help hold the cleansing bar together. In addition, colloidal oatmeal acts as an anti-itch agent (i.e., anti-pruritic).

In other preferred forms the bar has less than 2% by weight soap (e.g. is soap free), has 6-11% by weight of cetyl alcohol, the grain is a colloidal oatmeal available from Quaker Oats, and the surfactant is an isethionate surfactant (e.g. preferably an n-acyl isethionate salt). We prefer sodium cocoyl isethionate from PPG Industries (e.g. a formulation sold under the trade name "Jordapon CI" powder).

Other non-soap surfactants may be included such as anionic surfactants, nonionic surfactants, cationic surfactants, and zwitterionic surfactants. Linear alkyl sulfonates such as those described in Hawley's Condensed Chemical Dictionary, 12th Ed., Van Nostrand Reinhold Co., N.Y., p. 37 (1993) are particularly suitable and are referred to in the art by the name "syndet".

Preferred lathering enhancers are laureth-11-carboxylic acid, trideceth-7-carboxylic acid, ceteth-13-carboxylic acid, and trideceth-19-carboxylic acid. Preferred salt forms of the lathering enhancer are Na⁺, K⁺, TEA⁺, DEA⁺, MEA⁺, or NH₄⁺.

The bar can contain water (preferably 9-20%), and has a pH of between 5.0 and 7.0, preferably between 5.0 and 6.5, and even more preferably between 5.3 and 5.8. It thus has a pH similar to typical skin pH.

In another aspect, the invention provides a method of cleaning a human skin surface. The first step is to rub the above cleansing bar on the skin surface in the presence of water. The next step is to rinse the surface with additional water.

Other conventional additives can be included such as moisturizers (especially hardened vegetable oil and vegetable shortening) , humectants (e.g. glycerin), emollients, pigments, binding agents, neutralizers, foam stabilizers, preservatives, and buffering agents (such as lactic acid and sodium lactate) to offset any base content of the above compounds.

The objects of the present invention therefore include providing a processed grain based cleansing bar of the above kind which:
(a) does not require substantial amounts of soap;
(b) provides superior lathering characteristics;
(c) cleans at skin pH;
(d) is easy to rinse off; and
(e) has other desirable cleansing bar properties.

These and still other objects and advantages of the present invention (e.g. methods for using such cleansing bars) will be apparent from the description which follows. The description is merely of the preferred embodiments. The claims should be looked to in order to understand the full scope of the invention.

### Detailed Description

Three preferred cleansing bars of the present invention (A, B, C) and a prior art bar (D) contain the following ingredients:

| Ingredient | A(%) | B(%) | C(%) | D(%) |
|---|---|---|---|---|
| colloidal oatmeal - soothing agent, skin protectant | 38.00 | 38.00 | 38.36 | 38.00 |
| sodium cocoyl isethionate - synthetic detergent cleanser | 23.00 | 27.00 | 26.85 | 31.00 |
| purified water - forming aid | 9.71 | 14.59 | 14.59 | 14.40 |
| cetyl alcohol - moisturizer, binding agent | 7.20 | 8.00 | 8.58 | 8.50 |
| vegetable shortening, hardened - occlusive moisturizer | 4.50 | -- | -- | -- |
| hydrogenated vegetable oil - occlusive moisturizer | 4.50 | -- | -- | -- |
| laureth-11-carboxylic acid - lathering enhancer | 3.00 | 4.00 | 4.04 | -- |
| sodium hydroxide, anhydrous - neutralize AEC | 0.09 | 0.16 | 0.16 | -- |
| glycerin - humectant | 3.00 | 3.00 | 2.52 | 1.50 |
| cocamidopropyl betaine - foam stabilizer | 2.00 | -- | -- | -- |
| sodium lactate - buffer | 1.75 | 1.90 | 1.89 | 1.90 |
| cocamidopropyl phosphatidyl PG-dimonium chloride - cleansing agent emollient | 1.00 | 1.00 | -- | -- |
| titanium dioxide - whitening pigment | 0.95 | 0.95 | 0.96 | 0.95 |
| magnesium aluminum silicate - binding agent | -- | 0.50 | 0.50 | 0.50 |
| lactic acid - buffer | 0.50 | 0.30 | 0.30 | 1.70 |
| potassium sorbate - preservative | 0.50 | 0.30 | 0.50 | 0.50 |
| iodopropynyl butylcarbamate, 10% - preservative | 0.30 | 0.30 | 0.30 | 0.30 |
| odor masker | -- | -- | -- | 0.45 |
| moisturizer | -- | -- | 0.45 | 0.45 |

In the last example, the moisturizer of choice is PEG-14M, a polyethylene glycol polymer with 14,000 ethylene oxide units.

These cleansing bars can be made in a variety of ways. In one manufacturing process, the powders are added dry to a mixer capable of handling powders and blended to uniformity. Then, the liquid materials and aqueous solutions are added to the powders and blended.

Organic, waxy materials are first melted and then added as liquids (cetyl alcohol, hydrogenated vegetable oil, and hardened vegetable shortening). The batch is maintained at a temperature that keeps the blend in a more flowable (plastic) state (e.g. about 52°C). The uniformly blended mix is then discharged into standard bar forming equipment to be de-aerated, extruded, and pressed into bars.

If desired, one can pre-blend all the aqueous solutions and dispersible compounds, except for the sodium cocoyl isethionate. In addition, the alcohol ethoxycarboxylate (which is often provided in its acid form) is neutralized in the liquid pre-mix to form its sodium salt. This neutralization step could be carried out separately and the resulting aqueous salt solution could then be used in the aqueous premix.

While various test protocols were tried in connection with this invention, a typical one was as follows. Approximately 80 women who were at least 18 years of age were selected. The panelists were instructed to cleanse their whole face using their hands, at least once in the morning and, if applicable, at least once at night. Alternatively they were instructed to cleanse their hands with the test product, at least once in the morning and, if applicable, at least once at night.

They were told to use water that was "comfortably warm" in their cleansing process. Test samples were identified with code numbers. Performance and cosmetic attributes (including lathering) of the products were rated on a 7-point scale. For example, formulations C and D were compared.

In some tests, consumers were selected who were already familiar with oatmeal based bars. In other tests this was not a selection criteria. In some tests each consumer rated both a bar of the present invention and a prior art bar. In other tests random groups of consumers tested only one bar.

The testing established that consumers who were already familiar with prior art oatmeal based bars noted a significant lathering difference (with the present invention's bars having significantly better lathering). Interestingly, consumers not previously familiar with oatmeal based cleansing bars did not note this difference in a significant way. It is believed that this probably is due to such consumers subconscious comparisons to soap type bars. In any event, the present invention appears clearly advantageous for those consumers who accept and are familiar with the basic attributes of an oatmeal based bar.

While the present invention has been described with respect to formulations using sodium cocoyl isethionate and cetyl alcohol, other surfactants and fatty alcohols may be used in place of or in combination with those ingredients. For example, other acyl isethionate salts may be employed in place of or in combination with sodium cocoyl isethionate. Furthermore, other higher fatty alcohols, including, by way of example, myristyl alcohol and stearyl alcohol, may be employed in place of or in combination with cetyl alcohol.

While the present invention has been described with respect to the preferred embodiments, the invention is not limited to the disclosed embodiments. To the contrary, the present invention is intended to cover various modifications and equivalent arrangements. The following claims are thus to be accorded a broad interpretation, so as to encompass all such modifications.

### Industrial Applicability

The present invention provides cleansing bars and methods for using them.

## Claims

1. A cleansing bar, comprising:
at least 30% by weight of processed grain;
at least 10% by weight of a surfactant which is not a soap; and
at least .5% by weight of a lathering enhancer of the formula:
R-O-(CH₂CH₂O)ₙ-CH₂-COOH,
or a salt thereof,
wherein R is selected from the group consisting of alkyl moieties having between 8 and 20 carbons and alkyl aryl moieties having between 8 and 20 carbons, and n is 1-20.

2. The cleansing bar of claim 1, wherein the bar is less than 2% by weight soap and has at least 6% by weight cetyl alcohol.

3. The cleansing bar of claim 1, wherein the processed grain is colloidal oatmeal.

4. The cleansing bar of claim 1, wherein the surfactant is an isethionate surfactant.

5. The cleansing bar of claim 1, wherein the lathering enhancer is selected from the group consisting of laureth-11-carboxylic acid, trideceth-7-carboxylic acid, ceteth-13-carboxylic acid, trideceth-19-carboxylic acid and salts thereof.

6. The cleansing bar of claim 1, wherein the bar contains water and has a pH of between 5.0 and 7.0.

7. The cleansing bar of claim 6, wherein the bar has a pH of between 5.0 and 6.5.

8. The cleansing bar of claim 6, wherein the bar has a pH of between 5.3 and 5.8.

9. A method of cleaning a human skin surface, comprising:
rubbing the cleansing bar of claim 1 on the skin surface in the presence of water; and
then rinsing the surface with additional water.
